# EUROPEAN PATENT APPLICATION

(11) **EP 4 111 973 A1**
(43) Date of publication of application: **04.01.2023**
(21) Application number: 21461564.3
(22) Date of filing: 30.06.2021
(51) Int. Cl.: A61B 5/25, A61B 5/271, A61B 5/274, A61B 5/277, A61B 5/282, A61B 5/053

(54) **ELECTRODE ARRANGEMENT AND ELECTRODE SET FOR ELECTROCARDIOGRAPHIC AND/OR BIOIMPEDANCE MEASUREMENTS**

(71) Applicant: Wojskowa Akademia Techniczna, 00-908 Warszawa (PL); Wojskowy Instytut Medyczny, 04-141 Warszawa (PL)
(72) Inventor: WALCZAK, Andrzej, 00-908 Warszawa (PL); MARCINIAK, Piotr, 00-908 Warszawa (PL); BACZYK, Piotr, 00-908 Warszawa (PL); KRZESINSKI, Pawel, 04-141 Warszawa (PL); MURAWSKI, Piotr, 04-141 Warszawa (PL)
(74) Representative: Wlasienko, Jozef

(57) **Abstract**

The object of the present invention is an electrode system (100) for electrocardiographic and/or bioimpedance measurements and an electrode set (200) comprising the electrode system (100).

An electrode system (100) for electrocardiographic and/or bioimpedance measurements comprises at least a measuring electrode (110), which comprises a connector element (190) for connecting an electrical signal wire and a sensor element (195) for receiving measurement signals from the patient's body. Furthermore, electrode system (100) comprises a support element (115) comprising at least one electrode opening (120) which is provided with coaxial support attachment means (130) for attaching the measuring electrode (110) thereto, the measuring electrode (110) being provided with coaxial electrode attachment means (150) for pivotally mounting it in the electrode opening (120) of the support element (115) on the coaxial support attachment means (130), ensuring adjusting its position to the shape of the substrate to which it is applied.

## Description

### Description

### Field of the Invention

The object of the present invention is an electrode system and an electrode set for electrocardiographic and/or bioimpedance measurements. The electrode system and the electrode set according to the invention are intended to be reusable and may be used in a hospital and/or home conditions. The invention may find application in particular for impedance cardiography measurements.

### Background of the Invention

Bioimpedance methods such as impedance cardiography (ICG) and electrocardiography are the primary methods for the early detection and diagnosis of cardiovascular diseases. The primary measuring sensor in these methods are electrodes, which, depending on the method, are arranged in different places on the patient's body. Consequently, the patient's anatomical features significantly affect the accuracy of the electrode contact with the patient's skin. This is particularly important in the case of impedance cardiography measurements, in the case of which the electrodes are arranged in pairs at the base of the neck and on the midaxillary line of the xiphoid process.

Due to ergonomics and hygiene rules in hospital and home conditions, disposable electrodes are used to carry out measurements, especially impedance cardiography measurements. However, the technical problems associated with the use of electrodes such as detachment from the patient's skin, the high cost of using disposable electrodes, and the increasing concern for the environment necessitate the search for alternatives in the form of reusable electrodes.

### State of the Art

Various reusable electrode systems for use in bioimpedance and electrocardiographic measurements are well known in the art and are commercially available. These are, for example, clamp electrodes, suction cup electrodes or lamellar electrodes. However, due to the anatomical diversity of body parts, electrodes are usually dedicated to specific applications. For example, clamp electrodes are designed to pick up electrical signals from the extremities.

Additional technical problems arise when the use of multi-electrode leads is required in electrocardiographic and/or bioimpedance measurements. In the case of lamellar electrodes, rubber straps with holes are used to lead out connector elements and to position the electrodes on the patient's body. However, such a solution cannot be used when it is necessary to attach the electrodes at the base of the neck, especially for anatomical variations, such as, for example, a short neck, a wide neck, a slim neck with a deep "clavicular fossa", etc.

Moreover, a general problem with the variable shape of the anatomical parts of the patient's body is to achieve good electrical contact for the reusable electrodes. Attempts have been made to solve this problem by a reusable device that is disclosed in the international application WO2017096694A1. This application discloses a device for monitoring the cardiovascular system. The device comprises a holder on which electrodes are disposed, each electrode comprises a conductive portion and being placed on a conductive spring to facilitate adequate contact with the substrate, and arranged in a conductive spring seat and the mounting sleeve. However, like the reusable electrodes already mentioned, the device is dedicated and requires the use of an external force, such as, for example, manual force to press the electrodes against the patient's body. Therefore, the above device is not a universal solution for use in a variety of bioimpedance measurement methods.

Additionally, especially when reusable electrodes are used in a hospital conditions, for many patients, the necessary condition is that the device can be easily disinfected and the required sanitary conditions are maintained. For electrodes and/or devices of a complex structure, compliance with the required hygienic conditions becomes very difficult or even impossible.

Therefore, there are still unresolved technical problems in the prior art, which can include:
- a lack of versatility of reusable electrodes leading to dedicated structural solutions;
- instability or difficulty in achieving good electrical contact of the electrodes with the patient's body;
- a complex structure, preventing the compliance with the required hygienic conditions.

The present invention in the form of an electrode system and an electrode set for electrocardiographic and/or bioimpedance measurements of the claimed structure and application solves the above technical problems.

### Summary

The object of the present invention is to ensure a completely new solution that provides an electrode system and an electrode set for electrocardiographic and/or bioimpedance measurements.

In accordance with one aspect of the present invention, the present invention provides An electrode system for electrocardiographic and/or bioimpedance measurements, comprising at least one measuring electrode which comprises a connector element for connecting an electrical signal wire and a sensor element for receiving measurement signals from the patient's body, characterized in that it comprises a support element comprising at least one electrode opening which is provided with coaxial support attachment means for attaching the measuring electrode thereto, the measuring electrode being provided with coaxial electrode attachment means for pivotally mounting it in the electrode opening of the support element on the coaxial support attachment means, ensuring adjusting its position to the shape of the substrate to which it is applied.

Preferably, the support element comprises three electrode openings for mounting the measuring electrodes therein, respectively.

Preferably, the axes passing through the coaxial supporting attachment means of each of the three electrode openings form with each other an angle of substantially 60°.

Preferably, the connector element is a snap-fit socket for the electrical signal wire.

Preferably, when the electrode system comprises more than one measuring electrode, the connector elements of the measuring electrodes are electrically connected in parallel, so that each measuring electrode has a branch of the same electrical signal wire which measures the same electric potential at each measuring electrode.

According to a further aspect of the present invention, the present invention provides an electrode set for electrocardiographic and/or bioimpedance measurements, characterized in that it comprises an electrode system according to the invention and further comprises at least one positioning element and a housing for the electrode system for attaching the electrode system thereto, the housing of the electrode system being provided with engaging means for attaching the positioning element.

Preferably, the positioning element has an adjustable length and pressing force.

Preferably, the positioning element is a flexible retaining half-ring and/or an elastic band.

Preferably, the flexible retaining half-ring has the housing of the electrode system attached at both its ends.

Preferably, the flexible retaining half-ring is made in the form of a resilient bow for pressing the housing for the electrode system along with the electrode system attached thereto against the patient's body.

### Detailed Description of the Invention

An electrode system for electrocardiographic and/or bioimpedance measurements according to the invention typically comprises at least a measuring electrode, which comprises a connector element for connecting an electrical signal wire and a sensor element for receiving measurement signals from the patient's body.

The measuring electrodes used for measurements in the electrode system according to the invention comprise a substantially round and substantially flat sensor element, one side of which is intended to come into contact with the patient's skin and the other outer side of which has a connector element.

Commercially available electrodes approved for medical use can also be used as a starting element for the measuring electrode according to the invention. However, a shape other than the round shape of the sensor element of the measuring electrode can be envisaged. The electrode may be substantially flat, which should be understood as having a convex shape, for example the shape of a sphere sector or a spherical cup. A person skilled in the art will readily select the appropriate shape and size of the sensor element of the measuring electrode in accordance with the assumptions made and the requirements of the measuring method.

The connector element may be any required connector element that is compatible with the electrical signal wire connections of the electrocardiographic and bioimpedance measuring device. Preferably, the connector element is a snap-fit socket for the electrical signal wire.

The connector element may be arranged substantially arbitrarily, but preferably the connector element is arranged in a central part of the outer side of the sensor element of the measuring electrode and its diameter is smaller than the diameter of the sensor element of the measuring electrode, and more preferably the diameter of the connector element is at least two times smaller than that of the sensor element of the measuring electrode. Preferably, the diameter of the sensor element is from 5 mm to 9 mm. Preferably, the diameter of the connector element is suitably 2.5 mm. The electrode may preferably be made of a conductive material, preferably a corrosion-resistant one. Preferably, the electrode is made of a corrosion-resistant metal. The electrode is preferably made of duralumin, more preferably of PA9 zinc duralumin. Preferably, the material of the measuring electrode has a tensile strength of 350 MPa to 450 MPa, more preferably 400 MPa to 450 MPa.

According to the invention, the electrode system further comprises a support element comprising at least one electrode opening. The support element can have substantially any shape. Preferably, it is a flat or profiled element to adapt to the shape of a part of the patient's body and it comprises at least one electrode opening. Preferably, the support element is substantially round in shape or is substantially oval. Preferably, the support element is substantially round in shape or is substantially flat. Preferably, the support element has a diameter selected such that the required number of electrode openings can be arranged therein. The thickness of the support element depends on a material and method of the manufacture, as well as on structural assumptions and purpose of the electrode system. Preferably the support element is 1 mm thick, more preferably is less than 1 mm thick.

Furthermore, the support element may be made of any medically acceptable material, preferably a non-conductive material. It is preferably a polymeric material. Preferably, the support element is made of a rigid polymeric material, preferably with a modulus of elasticity between 3 GPa and 4 GPa, more preferably substantially equal to 3.6 GPa (measured according to DIN 500014, on a dry sample, at 23 °C). Preferably, the support element is made of polyimide.

According to the invention, the electrode opening may have any shape. Preferably, the shape of the electrode opening is complementary to that of the electrode. Preferably, the diameter of the support element opening is larger than that of the electrode sensor, so that the electrode sensor can be arranged in the electrode opening.

Moreover, according to the invention, the electrode opening is provided with coaxial support attachment means for attaching the measuring electrode thereto, and the measuring electrode is provided with coaxial electrode attachment means for mounting it pivotally in the support element electrode opening on the coaxial support attachment means. It is to be understood, that the coaxial support attachment means and the coaxial electrode attachment means are complementary and may be of any kind, so that once the electrode is attached in the measurement opening, these attachment means together ensure that the position of the electrode, in particular the electrode sensor, is adapted to the shape of the substrate it is applied to. Adaptation of the position of the electrode, in particular of the electrode sensor, to the shape of the substrate to which it is applied takes place by tilting the electrode, which is made possible by mounting it pivotally in the electrode opening of the support element by means of the complementary coaxial support attachment means and coaxial electrode attachment means.

The coaxial support attachment means and the coaxial electrode attachment means are complementary, and may be of any type, and together allow rotation to the extent required. Preferably, the coaxial support attachment means and the coaxial electrode attachment means allow full rotation. In order to adapt the position of the electrode to the substrate, the electrodes preferably pivot out of the plane of the electrode opening or a plane parallel to the plane of the electrode opening, where the plane of the electrode opening is an abstract plane in which the electrode opening is contained. Moreover, arbitrarily selected structural elements of the electrode system may restrict full rotation so that the electrode is free to tilt for adapting to the substrate to which it is applied.

Preferably, the coaxial support attachment means and the coaxial electrode attachment means are coaxial with the diameter of the electrode opening.

The coaxial support attachment means and the coaxial electrode attachment means are complementary. Preferably, the coaxial support attachment means and the coaxial electrode attachment means are complementarily provided as an axle or a half-shaft or any kind of a rod, and an opening or a receiving element complementary therewith, such that rotation of the electrode in the electrode opening is ensured. Preferably, the electrode opening may be provided with two coaxial half-shafts which constitute the coaxial support attachment means, and the electrode is provided with at least one element with a receiving hole for the half-shaft, preferably two such elements with a receiving hole for the half-shaft, constituting the coaxial electrode attachment means. Preferably, the electrode may be provided with two coaxial axles constituting the coaxial electrode attachment means, and the electrode opening is provided with at least one element with a receiving hole for the half-shaft, preferably two such elements with a receiving hole for the half-shaft, constituting the coaxial support attachment means. Moreover, preferably the electrode opening may be provided with an axle which constitutes the coaxial support attachment means, and the electrode is provided with at least one element with a receiving hole for the axle, constituting the coaxial electrode attachment means. Preferably, the electrode may be provided with an axle which is the coaxial electrode attachment means, and the electrode opening is provided with at least one element with a receiving hole for the half-shaft, constituting the coaxial support attachment means.

Moreover, the coaxial support attachment means and the coaxial electrode attachment means can be made of any material. For example, when the electrode opening is provided with two coaxial half-shafts which constitute the coaxial support attachment means, they may be made of the same material as the support element. For example, the half-shafts, which constitute the coaxial support attachment means, may be made of the same material as the support element and have the shape of a pin, axle or half-shaft. Also the coaxial electrode attachment means can be made of the same material as the electrode, or the coaxial electrode attachment means can be made of a different material than the electrode.

In the basic position, the surface of the sensor element and/or the surface defined by the circumference of the sensor element is substantially parallel to the plane of the electrode opening. Preferably, in the basic position, the surface of the sensor element is arranged above the plane of the electrode opening, in other words it projects above the plane of the electrode opening. Preferably, in the base position, the surface of the sensor element is arranged from 0.1 mm to 1 mm, more preferably 0.3 mm to 0.7 mm, even more preferably 0.5 mm above the plane of the electrode opening. The specified values are, however, the preferred values and other values are possible which result from the shape of the measuring substrate.

Considering the above, a person skilled in the art will readily select dimensions, shapes and materials for the electrode, support element, coaxial support attachment means and coaxial electrode attachment means, as well as the dimensions and shape of the electrode openings, as well as methods of the manufacture thereof, which are commonly known methods in the prior art, including also 3D printing.

The electrode system preferably comprises at least one electrode and at least one electrode opening. The electrode system preferably comprises two or three, or four electrodes and two or three, or four electrode openings, respectively. The electrode system may comprise more electrodes and correspondingly more electrode openings, preferably the number of electrode openings is greater than or equal to the number of electrodes.

Preferably, the electrode system comprises three electrode openings for mounting three measuring electrodes, respectively. Such an electrode system is particularly preferable because it implements the principle of mechanical statics of any system based on three points, and thus allows the effective adaptation and contact of the electrodes of the electrode system with the patient's body.

The electrode openings, electrodes, coaxial support attachment means and coaxial electrode attachment means can be arranged arbitrarily in the support element depending on the requirements of the measurement method. Preferably, the electrode openings are arranged in the support element substantially equidistantly from the adjacent electrode opening. In this way the most compact electrode system based on regular figures can be created.

The axes passing through the coaxial support attachment means which likewise extend through the coaxial electrode attachment means, may make any angles with each other. They can, for example, be parallel to each other. For example, some of the axes may be parallel and some may be perpendicular to each other. Preferably, these axes make angles of regular polygons with each other. Preferably, for an electrode system comprising three electrode openings and electrodes, the axes passing through the coaxial support attachment means of each of the three electrode openings make an angle of substantially 60° with each other. Preferably, for an electrode system comprising four electrode openings and electrodes, the axes passing through the coaxial support attachment means of each of the three electrode openings make an angle of substantially 90° with each other.

Another problem indicated in the prior art is the deterioration or complete loss of contact of the electrode with the patient's skin during the measurement as a result of changes in the patient's position or motion, which causes distortion or disturbance of the measured signal, thus leading to a misinterpretation of the measurement results or the need to repeat measurements. Although the structure of the electrode system according to the invention significantly reduces the risk of loss or deterioration of contact by each of the electrodes, but also in this case undesirable disturbances in the measurements can occur. In the electrode system according to the invention comprising more than one electrode, each electrode is provided with an independent connector element. This means that within the electrode system, each measuring electrode can be freely connected in accordance with the accepted requirements. In order to compensate for measuring errors resulting from the reading from a single measuring electrode, the measuring electrodes preferably comprise connector elements electrically connected in parallel, so that each measuring electrode has a branch of the same electrical signal wire which measures the same electric potential at each measuring electrode. This results in much greater measurement stability. Preferably, the connector element is a snap-fit element.

The present invention also allows greater versatility in the use of the electrode system according to the invention for measurements in various leads and parts of the patient's body through the claimed electrode set comprising the electrode system according to the invention. The electrode set according to the invention comprises at least one positioning element and a housing for the electrode system for attaching the electrode system therein, the housing of the electrode system being provided with engaging means for attaching the positioning element.

The housing may be of any shape so that it is possible to attach the electrode system therein and to provide connection for the electric wires to lead out the measurement signals. Preferably, the housing may have the shape of a cylinder with an oval or round base and a diameter matching the diameter of the support element. The upper part may be an electrode system, and the housing base, which is a part of the housing for the electrode system, may comprise means for leading out electric wires and engaging means.

Furthermore, the housing for the electrode system can be made of any material approved for medical use. Preferably, the housing for the electrode system is made of the same material as the support element.

The positioning element may be any element capable of disposing the housing for the electrode system on the patient's body. It may be, for example, a tape with an adhesive element. Preferably, the positioning element has an adjustable length and pressing force. Preferably, the positioning element is a flexible retaining half-ring and/or an elastic band. More preferably, the positioning element is a flexible retaining half ring with an adjustable length and pressing force and/or an elastic band with an adjustable length and pressing force. Preferably, the retaining half-ring is made in the form of a resilient bow for pressing the housing for the electrode system along with the electrode system attached thereto against the patient's body. Preferably, the flexible retaining half-ring with an adjustable length allows to adjust the position of the electrode system attached in the housing for the electrode system, adjusting its length to the size of the patient's body. In addition, the flexible retaining half-ring can be made as a flat spring, which presses the electrodes against the patient's neck with an adjustable pressing force, which allows to maintain a constant contact between the measuring electrodes and the patient's skin. Furthermore, the positioning element can also be an elastic band, such as, for example, a rubber band.

At least one housing for the electrode system can be arranged on the positioning element by means of engaging means. Preferably, depending on the needs, two housings for the electrode system or even more housings for the electrode system can be arranged on the positioning element.

The arrangement of the housing for the electrode system on the positioning element can be arbitrary. Preferably, the positioning element at both its ends has a housing of the electrode system attached. In particular, the flexible retaining half ring has housing of the electrode system attached at both its ends. The flexible retaining half ring may have the housing of the electrode system attached at one end only.

The engaging means may be any suitable engaging means for attaching and arranging the housing for the electrode system on the positioning element. The engaging means may be detachable engaging means and non-detachable engaging means, where detachable engaging means being preferred. Moreover, the engaging means may preferably enable changing the position of the housing, and therefore the electrode system, in relation to the patient's body and/or the positioning element. The engaging means may comprise, but are not limited to, clasps, clips, latches, systems that enable pivoting of hinge connections type, systems comprising a pivoting axle and axle receiving means, etc.. A person skilled in the art will readily select particular components and materials to provide the engaging means suitable for connecting the housing to the positioning element. Engaging means which allow pivoting or rotating movement of the housing for the electrode system are preferred.

Moreover, as used herein, the term "diameter" is intended to mean the diameter of a set, which is the supremum of the distance of all pairs of points for a limited subset of a metric space, that is, of any figure or section. Unless otherwise stated, when all quantities are defined by numerical values and ranges, ranges resulting from any combination of their values provided herein and values falling within the given ranges are also preferred.

A person skilled in the art will readily select particular structural elements, dimensions, materials without departing from the scope of the invention, and specific implementations of the invention will be illustrated in non-limiting embodiments.

The solution provided according to the technical features proposed above enables:
- providing versatility of reusable electrodes for measurements on different leads and body parts;
- obtaining stability of the contact of the measuring electrode with the patient's body;
- maintaining the required hygienic conditions with repeated use due to the structure of the electrode system.

For example, it is worth noting that the good contact with the patient's body is achieved by using a new structure of the electrode system and a pivotable suspension of the measuring electrode in the support element, so that it becomes possible to easily adapt the sensor element of the measuring electrode to the substrate to create an electrical contact therewith.

### Brief description of the drawing

The object of the present invention is disclosed in the embodiments with reference to the attached drawing, in which:
- FIG. 1A: is a top oblique view of the electrode system with measuring electrodes attached;
- FIG. 1B: is a bottom oblique view of the electrode system with measuring electrodes attached;
- FIG. 2A: is a schematic top view of the electrode system with a measuring electrode attached;
- FIG. 2B: is a cross-section of the electrode system with a measuring electrode attached;
- FIG. 3: is an electrode set according to the first embodiment without measuring electrodes attached;
- FIG. 4: is a schematic representation of the electrode set shown in Fig. 3 positioned on the patient's neck as a component for ICG measurements;
- FIG. 5A: is an oblique view of a housing for the electrode system of an electrode set of the second embodiment;
- FIG. 5B: is a side view of a housing for the electrode system of an electrode set of the second embodiment;

### Embodiments of the Invention

Herein below, object of the present invention is described in detail with reference to the accompanying Figures and embodiments. Figures are illustrative of the invention and to understand the invention better, have been made without precisely keeping to the scale and aspect ratio of the elements depicted therein. The present invention is not limited to the specific embodiments described herein.

### First Embodiment of the Invention

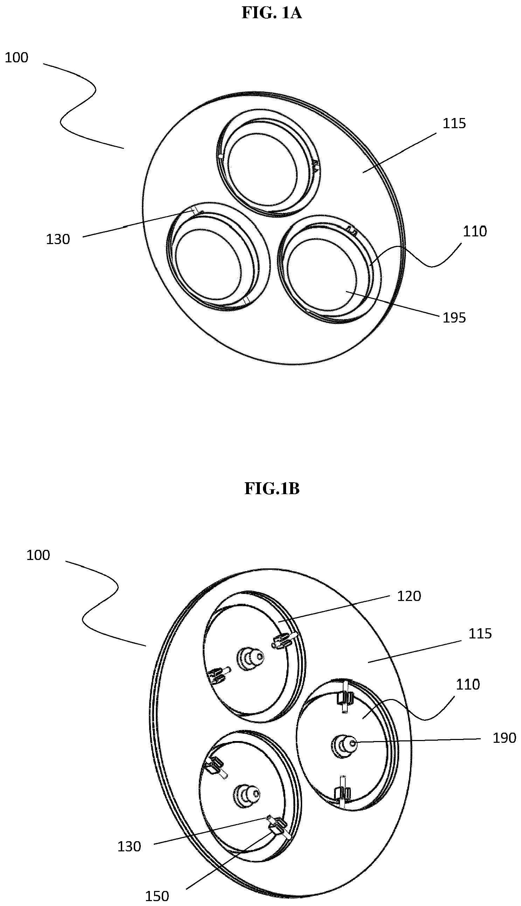

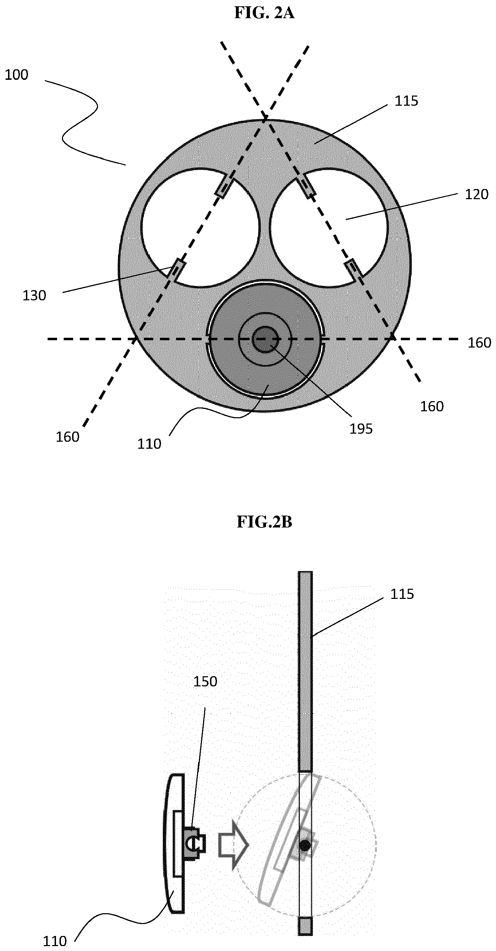

In the first embodiment of the invention, the electrode system 100 according to the invention is shown. The electrode system according to the first embodiment is shown in Fig. 1A, Fig. 1B, Fig. 2A and Fig. 2B.

Fig. 1A is a top oblique view of the electrode system 100 with the measuring electrodes 110 attached showing the sensor elements 195 of the measuring electrodes 110 mounted in the support element 115. According to the first embodiment, the electrode system 100 has three measuring electrodes 110 attached in the support element 115. In contrast, Fig. 1B is a bottom oblique view of the electrode system 100 with the measuring electrodes 110 attached, so that connector elements 190 of the measuring electrodes 110 on the opposite side of the sensor elements 195 of the measuring electrodes 110 are shown. The round measuring electrodes 110 are positioned in the round electrode openings 120 of the round support element 115.

The measuring electrodes 110 are positioned in the electrode openings 120 of the support element 115 by means of the mutually complementary coaxial support attachment means 130 and coaxial electrode attachment means 150. The coaxial support attachment means 130 were made in the form of half-shafts (pins), while the coaxial electrode attachment means 150 were made in the form of snap-fit elements with an opening for receiving the pin of the coaxial support attachment means 130, while allowing the measuring electrode to rotate.

According to the first example, as shown in Fig. 2A, the three electrode openings 120 form an equilateral triangle. The axes 160 of the coaxial support attachment means 130 and the coaxial electrode attachment means 150 of each of the electrodes form the 60° angle with each other. Such a structure of the electrode system is particularly preferred, because the measurement electrodes 110 together with the electrode openings 120 form the densest packing arrangement, thus with a 9 mm diameter of the sensor element 195 of the measurement electrode 110, an electrode system 100 with a support element diameter of 21 mm is obtained. Moreover, the axes 160 system implements the principle of mechanical statics of any system based on three points, and thus allows the effective adaptation and contact of the electrodes of the electrode system with the patient's body.

Fig. 2B is a cross-sectional view of an electrode system 100 with a measuring electrode attached. The dotted line in Fig. 2B shows the possibility of rotation of the electrode on the coaxial support attachment means 130, so that in case of uneven surfaces it is possible for the electrode to tilt and adapt to unevenness of the substrate.

The support element was made of rigid polyimide (with a modulus of elasticity at break of 3.6 GPa), and the electrodes were made of PA9 zinc duralumin.

### Second Embodiment of the Invention

The electrode system 100 of the second embodiment is shown in Fig. 1A, Fig. 1B, Fig. 2A and Fig. 2B, and is the same as the electrode system 100 of the first embodiment, except that according to the second embodiment, to the connector element 190, in the form of a snap-fit element as shown in Fig. 1B, connection wires (not shown) were electrically connected in parallel, so that each measuring electrode has a branch of the same electric signal wire that measures the same electric potential at each measuring electrode.

### Third Embodiment of the Invention

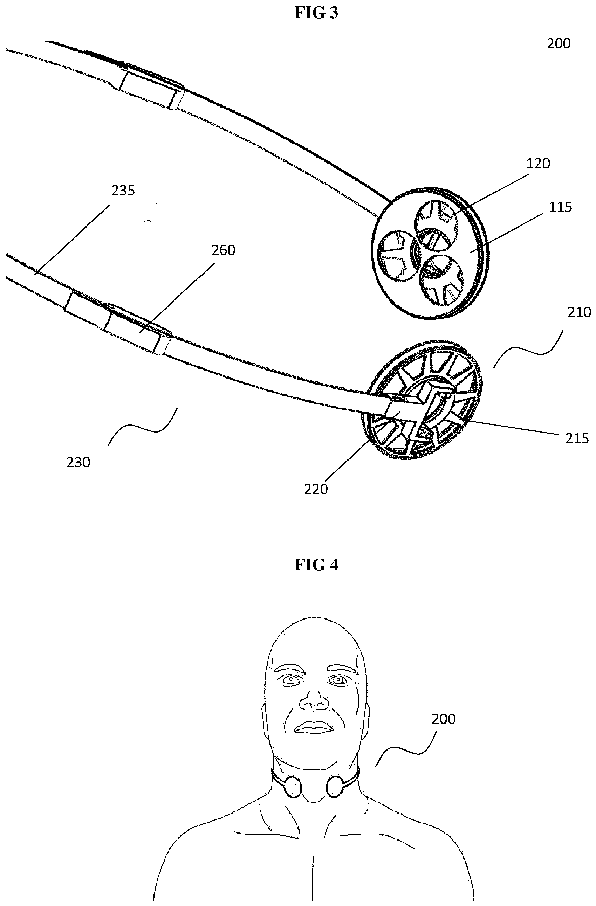

The third embodiment of the invention presents an electrode set 200. The electrode set according to the third embodiment is shown in Fig. 3 and Fig. 4. Fig. 3 shows an electrode set 200 comprising:
- the electrode system 100, where for the sake of clarity of Fig. 3, only the support element 115 of the electrode system 100 is shown;
- the housing 210 for the electrode system 100; and
- the positioning element 230.

The housing 210 is in a shape adapted to receive the electrode system 100. The housing base 215 is provided with the engaging means 220. The engaging means 220 in one part allow the housing 210 for the electrode system to be rotatable attached, and in the other part allows the positioning element 230 to be attached.

According to the third embodiment, the positioning element 230 is a flexible retaining half-ring 235 which is made in the form of a metal resilient bow for pressing the housing 210 for the electrode system together with the electrode system 100 against the patient's body. The flexible retaining half-ring 235 is provided with the adjusting element 260 as a through-clamp element so that the length of the positioning element 230 can be adjusted. According to this embodiment, the flexible retaining half ring 235 comprises three elements in a form of a flat metal spring such that the three flat spring elements connected by the two adjusting elements 260 form a metal resilient bow.

Fig. 4 shows the electrode set 200 of a third embodiment positioned on the neck of a patient as a part of an impedance cardiography (ICG) measurement kit.

### Fourth Embodiment of the Invention

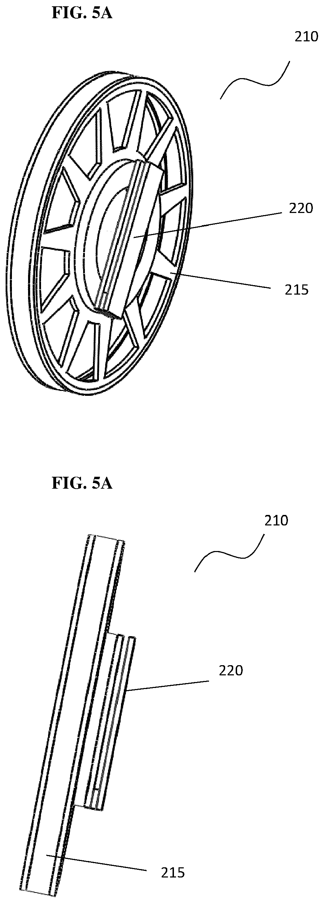

The fourth embodiment of the invention shows a part of the electrode set 200 in the form of a housing 210 for the electrode system 100. The housing 210 for the electrode system 100 according to the fourth embodiment is shown in Figs. 5A and 5B.

The housing 210 for the electrode system 100 according to the fourth embodiment comprises the base 215 of the housing 210 for the electrode system 100 and the engaging means 220. The engaging means 220 are formed as a flat engaging element. The engaging means 220 made as a flat engaging element are suitable for receiving a positioning element in the form of a length adjustable rubber elastic band (not shown).

The foregoing description of the presented embodiments is provided to enable a person skilled in the art to make and/or use the present invention, and is not intended to limit the scope of protection as defined by the claims. Since the presented description of the embodiments is not exhaustive or limiting, modifications and variations to the presented embodiments of the invention are also possible.

Any features, elements or components of any of the embodiments discussed above are claimed alone or in combination with any features, elements or components of any of the other embodiments discussed above and the features disclosed herein.

### LIST OF REFERENCE NUMERALS

- 100: electrode system
- 110: measuring electrode
- 115: support element
- 120: electrode opening
- 130: coaxial support attachment means
- 150: coaxial electrode attachment means
- 160: axes - an abstract element
- 190: connector element
- 195: sensor element
- 200: electrode set
- 210: housing for the electrode system
- 215: housing base
- 220: engaging means
- 230: positioning element
- 235: flexible retaining half ring
- 260: adjusting element

## Claims

1. An electrode system (100) for electrocardiographic and/or bioimpedance measurements, comprising at least one measuring electrode (110) which comprises a connector element (190) for connecting an electrical signal wire and a sensor element (195) for receiving measurement signals from the patient's body, **characterized in that** it comprises a support element (115) comprising at least one electrode opening (120) which is provided with coaxial support attachment means (130) for attaching the measuring electrode (110) thereto, the measuring electrode (110) being provided with coaxial electrode attachment means (150) for pivotally mounting it in the electrode opening (120) of the support element (115) on the coaxial support attachment means (130), ensuring adjusting its position to the shape of the substrate to which it is applied.

2. The electrode system according to claim 1, **characterized in that** the support element (115) comprises three electrode openings (120) for mounting the measuring electrodes (110) therein, respectively.

3. The electrode system (100) according to claim 1 or 2, **characterized in that** the axes (160) passing through the coaxial supporting attachment means (130) of each of the three electrode openings (120) form with each other an angle of substantially 60°.

4. The electrode system (100) according to any one of the preceding claims, **characterized in that** the connector element (190) is a snap-fit socket for the electrical signal wire.

5. The electrode system according to any one of the preceding claims, **characterized in that,** when the it comprises more than one measuring electrode (110), the connector elements (190) of the measuring electrodes (110) are electrically connected in parallel, so that each measuring electrode (110) has a branch of the same electrical signal wire which measures the same electric potential at each measuring electrode (110).

6. An electrode set (200) for electrocardiographic and/or bioimpedance measurements, **characterized in that** it comprises an electrode system (100) according to one of claims 1 to 5 and further comprises at least one positioning element (230) and a housing (210) for the electrode system for attaching the electrode system (100) thereto, the housing (210) of the electrode system being provided with engaging means (220) for attaching the positioning element (230).

7. The electrode set (200) according to claim 6, **characterized in that** the positioning element (230) has an adjustable length and pressing force.

8. The electrode set (200) according to claim 6 or 7, **characterized in that** the positioning element (230) is a flexible retaining half-ring (235) and/or an elastic band.

9. The electrode set (200) according to claim 8, **characterized in that** the flexible retaining half-ring (235) has the housing (210) of the electrode system attached at both its ends.

10. The electrode set (200) according to claim 8 or 9, **characterized in that** the flexible retaining half-ring is made in the form of a resilient bow for pressing the housing (210) for the electrode system along with the electrode system (100) attached thereto against the patient's body.
